# EUROPEAN PATENT APPLICATION

(11) **EP 4 844 772 A1**
(43) Date of publication of application: **25.11.2026**
(21) Application number: 24918803.8
(22) Date of filing: 16.01.2024
(51) Int. Cl.: G16B 50/00, G16B 20/20, G16B 30/10

(54) **GENOME INFORMATION PROCESSING METHOD, GENOME INFORMATION PROCESSING SYSTEM, AND GENOME INFORMATION PROCESSING PROGRAM**

(71) Applicant: NEC Corporation, 108-8001 Tokyo (JP)
(72) Inventor: TANAKA, Yuki, Tokyo 108-8001 (JP); ONOUE, Kousuke, Tokyo 108-8001 (JP); YOSHIHARA, Yoshiko, Tokyo 108-8001 (JP); YOKOTA, Haruki, Tokyo 108-8001 (JP)
(74) Representative: Betten & Resch
(86) International application number: PCT/JP2024/000895
(87) International publication number: WO 2025/154155

(57) **Abstract**

A genomic information processing system includes a company server that performs a series of processes up to identify location(s) containing mutation(s) in genomic data contained in a patient's specimen, and issues instructions to register and record a history of each of the processes on a blockchain; and a management apparatus that receives the instructions from the company server and registers and records the history of each of the processes on the blockchain.

## Description

### TECHNICAL FIELD

The present invention relates to a method for processing genomic information, a genomic information processing system, and a genomic information processing program.

### BACKGROUND ART

Traditionally, health care has been primarily disease-centered, with the main objectives being to identify the causes of diseases and develop treatments for them. While it has long been recognized that the same treatment is not equally effective for everyone, even for the same disease, this was difficult to address because individual variances in clinical outcomes could only be determined by observing the treatment and its effects, making it challenging to develop treatment plans optimized for each individual.

Meanwhile, advances in technologies such as DNA sequencing and the identification of single-nucleotide polymorphisms (SNPs) within DNA sequences have made it possible to observe how an individual's genes differ from those of others. Consequently, it has become practicable to use this information to select the most appropriate treatment for a specific patient or to develop and manufacture medicines optimized for an individual. For example, Patent Document 1 describes a method for obtaining highly reliable genomic variant information from read data obtained from a next-generation sequencer.

### CITATION LIST

### PATENT LITERATURE

[Patent Literature 1]
Japanese Patent Kokai Publication No. JP2017-016665A

### SUMMARY

### TECHNICAL PROBLEM

The disclosures in the above prior art documents are hereby incorporated by reference into this document. The following analyses were conducted by the inventors.

Meanwhile, genomic data representing the nucleotide sequences of DNA obtained from an individual is, in many cases, generally considered to constitute a "personal identification code" and therefore requires strict management as personal information. Furthermore, it is anticipated that in many cases, "genomic information"-which involves applying medical interpretations, such as information on mutations, to genomic data obtained from patients-will be treated as sensitive personal information requiring special care in its handling.

Furthermore, since such genomic data is considered personal information, it is not sufficient to merely protect it from leaks or tampering. Specifically, entities that receive genomic data must process it only in accordance with the consent given by the data provider. These entities are required not only to protect the data from leaks or tampering but also to strictly manage the processing they perform on the genomic data.

In view of the aforementioned issues, the objective of the present invention is to provide a genomic information processing method, a genomic information processing system, and a genomic information processing program that contribute to managing the history of each step in the series of processes involved in identifying locations containing mutations in a patient's specimen.

### SOLUTION TO PROBLEM

According to a first aspect of the present invention, there is provided a method for processing genomic information, wherein a history of each of a series of processes up to identifying location(s) containing mutation(s) in genomic data contained in a patient's specimen is registered and recorded on a blockchain.

According to a second aspect of the present invention, there is provided a genomic information processing system, comprising a company server that performs a series of processes up to identify location(s) containing mutation(s) in genomic data contained in a patient's specimen, and issues instructions to register and record a history of each of the processes on a blockchain; and a management apparatus that receives the instructions from the company server and registers and records the history of each of the processes on the blockchain.

According to a third aspect of the present invention, there is provided a genomic information processing program that causes a computer to perform process of registering and recording a history of each of the series of processes on a blockchain, upon receiving instructions from a company server, which performs the series of processes up to identify location(s) containing mutations in genomic data contained in a patient's specimen, to register and record the history of each of the series of processes on the blockchain. Further, this program can be stored in a computer-readable storage medium. The storage medium may be a non-transitory one such as a semiconductor memory, a hard disk, a magnetic recording medium, an optical recording medium, and the like. The present invention can also be realized as a computer program product.

### ADVANTAGEOUS EFFECTS OF INVENTION

According to each aspect of the present invention, a genomic information processing method, a genomic information processing system, and a genomic information processing program can be provided that contribute to managing the history of each step in a series of processes leading up to the identification of locations containing mutations in a patient's specimen.

### BRIEF DESCRIPTION OF THE DRAWINGS

[Fig. 1]

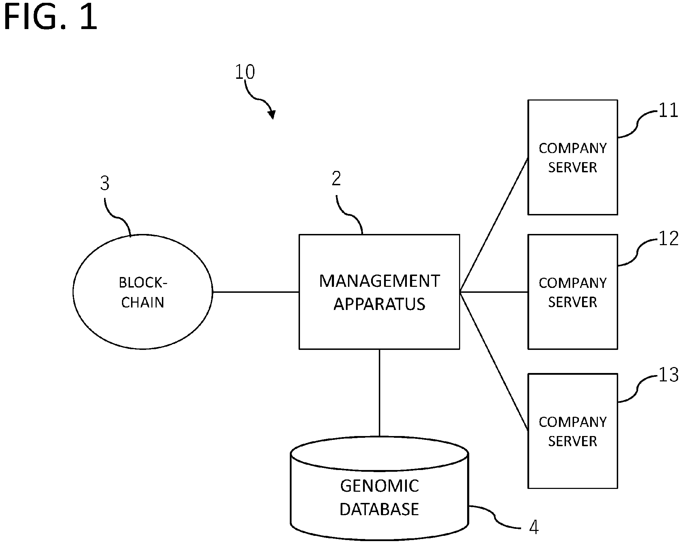

   Fig. 1 illustrates an example of the schematic configuration of a genomic information processing system.
[Fig. 2]

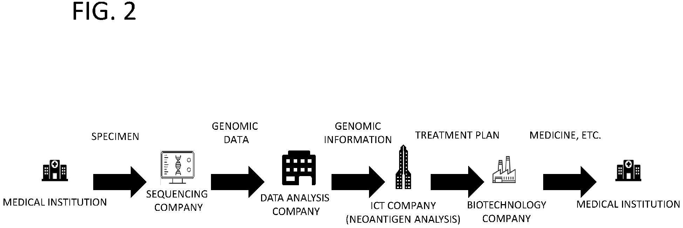

   Fig. 2 is a schematic diagram illustrating an example of the series of processes applied to genomic data in personalized medicine.
[Fig. 3]

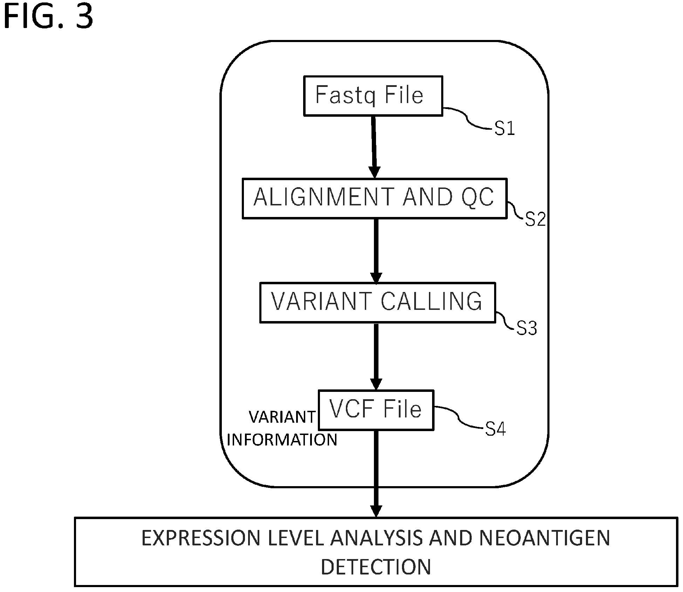

   Fig. 3 illustrates the series of steps involved in extracting variant information from genomic data.
[Fig. 4]

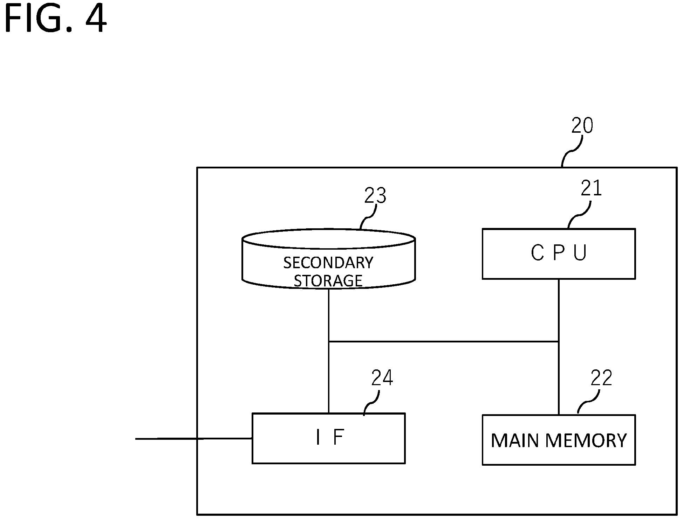

   Fig. 4 is a diagram illustrating an example of the hardware configuration of the company servers and management apparatus used in the embodiments.

### EXAMPLE EMBODIMENTS

The embodiments of the present invention will be described below with reference to the drawings. However, the present invention is not limited to the embodiments described below. Furthermore, in each drawing, identical or corresponding elements are denoted by the same reference numerals. It should also be noted that the drawings are schematic, and the dimensional relationships and proportions of the elements may differ from those in reality. It should also be noted that there may be portions within the drawings where the dimensional relationships or proportions differ from one another.

Fig. 1 illustrates an example of the schematic configuration of a genomic information processing system. As shown in Fig. 1, the genomic information processing system 10 comprises a plurality of company servers 11, 12, and 13, a management apparatus 2, a blockchain 3, and a genomic database 4.

A plurality of company servers 11, 12, and 13 perform a series of processes to identify the locations containing mutations in the genomic data included in the patient's specimen, and issue instructions to the management apparatus 2 to register and record the history of each of these processes on the blockchain 3. As will be described in detail later, the process of identifying locations containing mutations in the genomic data contained in the patient's specimens consists of a series of consecutive steps. Therefore, while it is conceivable to execute the entire series of processes on a single company server, it is also conceivable to distribute the execution among plural company servers 11, 12, and 13.

The management apparatus 2 receives instructions from a plurality of company servers 11, 12, and 13 and registers and records the history of each step in a series of processes on the blockchain 3. The genomic database 4 records genomic data obtained from the subject's specimen and mutation information obtained by performing a series of processes on the genomic data. Note that the genomic information processing system 10 shown in Fig. 1 employs a configuration comprising a plurality of company servers 11, 12, and 13 and a separate management apparatus 2; however, the management apparatus 2 may be implemented as part of the functions of the plurality of company servers 11, 12, and 13, and the system may be configured such that each of the plurality of company servers 11, 12, and 13 directly registers and records the history of each step in the series of processes-up to the point of identifying the locations containing mutations in the genomic data contained in the patient's specimens-on blockchain 3.

Fig. 2 is a schematic diagram illustrating an example of the series of processes applied to genomic data in personalized medicine. As shown in Fig. 2, personalized medicine is typically carried out by multiple companies sharing the responsibilities.

Specimens such as blood and tissue samples obtained from patients at medical institutions are sent to sequencing companies. Sequencing companies sequence the received specimens and obtain genomic data (read information) representing the nucleotide sequences of the genomes contained in the specimens. However, since genomic data (read information) is merely the nucleotide sequence of the genome, it cannot be used on its own to provide personalized medicine. Therefore, sequencing companies send the genomic data (read information) to data analysis companies.

Data analysis companies analyze genomic data (read information) to generate variant information that identifies the locations where variants are contained. Here, variant information refers to information regarding variants in the genomic nucleotide sequence, including the position of the nucleotide sequence containing the variant and the type of variant, such as a single-nucleotide variant, insertion, or deletion. This variant information relates to the individual patient's biological characteristics and diseases, making it crucial for personalized medicine. However, even when an individual patient's variant information is obtained, it is not sufficient to determine a treatment plan for personalized medicine. Therefore, data analysis companies send genomic information-genomic data annotated with variant information-to ICT (Information and Communication Technology) companies.

ICT companies perform neoantigen analysis using genomic data that includes mutation information to determine treatment strategies. Neoantigens are cancer antigens (markers) that have newly emerged due to genetic mutations in cancer cells, for example, and they provide critical information for determining personalized treatment strategies. Furthermore, since these genetic mutations do not necessarily occur whenever cancer cells appear, it is necessary to analyze them through neoantigen analysis of genomic data supplemented with mutation information. On the other hand, once neoantigen information is obtained, the neoantigens become targets for personalized medicine.

ICT companies send treatment plans derived from neoantigen analysis of genomic data-which includes mutation information-to biotechnology companies. Biotechnology companies use the results of this neoantigen analysis to manufacture appropriate medicines and other products. The medicines and other products manufactured by biotechnology companies are then sent to medical institutions, where they are used to provide personalized treatment to patients.

As noted, the processes described above are examples of a series of procedures applied to genomic data in personalized medicine; in actual practice, different procedures may be employed. For example, neoantigen analysis is an example of a procedure used in personalized medicine, and in some cases, cancer gene panel testing may be performed instead of neoantigen analysis. Cancer gene panel testing is a test that examines mutations in DNA sequences to identify medicines expected to be effective and information on clinical trials. When an ICT company performs cancer gene panel testing, it sends the information on medicines expected to be effective and clinical trials obtained from the test results to a biotechnology company. Biotechnology companies then manufacture medicines and other products suitable for the treatment plans derived from the cancer gene panel testing. These medicines and products, manufactured by the biotechnology companies, are sent to medical institutions, where they are used to provide personalized medicine to patients.

Therefore, in personalized medicine, the process involves not only obtaining genomic data-which consists solely of the nucleotide sequence of the genome-from a specimen, but also performing multiple subsequent steps. In particular, the process of generating genomic information by adding variant information to the genomic data is essential for deriving information useful for personalized medicine from the raw nucleotide sequence data. Furthermore, the process of generating genomic information by adding variant information to genomic data is not a single step but rather a series of processes consisting of multiple steps.

Fig. 3 illustrates the series of steps involved in extracting variant information from genomic data. As shown in Fig. 3, the process for extracting variant information from genomic data (read data) comprises: a process (S1) for sequencing the patient's specimens to generate read data (Fastq file), which represents the genomic nucleotide sequence; and a process (S2) for generating alignment information (Alignment) by mapping the read data to a reference sequence, and evaluating the quality of the alignment information (QC; quality control), and a process (S3) that detects variants from the alignment information and generates variant information (VCF file).

The process (S1) of generating read data (Fastq file) involves a sequencer reading the nucleotide sequence of the genome from the patient's specimen. The read data (Fastq file) is the output of the sequencer. The read data (Fastq file) contains the nucleotide sequence and its quality scores.

The process of generating alignment information (Alignment) for read data involves comparing the read data (Fastq file) with a reference sequence and aligning locations with matching sequences. The process (S2) of evaluating the quality of the alignment information (QC; quality control) involves removing locations with low sequence determination accuracy from the alignment information to ensure that subsequent analyses are not compromised.

In the process (S3) of detecting variant information from alignment information and generating variant information (VCF file), variants are detected from the alignment information, and variant information (VCF file) is output (S4). The variant information (VCF file; Variant Call Format file) contains information on mutations in the nucleotide sequence.

The variant call file (VCF file) obtained through the above series of steps is used for further analyses, such as neoantigen analysis.

As described above, the process of identifying locations containing mutations in genomic data consists of multiple steps. Therefore, it is possible for multiple companies to share the responsibilities for performing the process of identifying locations containing mutations in genomic data. In other words, as shown in Fig. 1, it is possible to have a genomic information processing system 10 equipped with multiple company servers 11, 12, and 13, where these servers 11, 12, and 13 share responsibilities to perform the process of identifying locations containing mutations in the genomic data.

A plurality of company servers 11, 12, and 13 perform at least a part of a series of processes described above up to identifying locations containing mutations in the genomic data, and send instructions to the management apparatus 2 to register and record the history of each of those processes on the blockchain. Here, the history of each process uses a hash value or a combination of hash values derived from a combination of the Specimen ID, Patient ID, processing date, processing program name, program version, and processing results (outputs, evaluation results). While it is preferable to register and record the processing history on the blockchain, it is also possible to include the hash value of the genomic data.

### (Example Hardware Configuration)

Fig. 4 is a diagram illustrating an example of the hardware configuration of the company servers and management apparatus used in the embodiments. Specifically, company servers 11, 12, and 13, and management apparatus 2 enable the realization of their respective functions by performing the genomic information processing method described above as a program on an information processing apparatus (computer) 20 that adopts the hardware configuration shown in Fig. 4. However, the hardware configuration example shown in Fig. 4 is merely one example of a hardware configuration for realizing the respective functions of company servers 11, 12, 13, and management apparatus 2, and is not intended to limit the hardware configuration of company servers 11, 12, 13, and management apparatus 2. Company servers 11, 12, 13, and management apparatus 2 may include hardware not shown in Fig. 4.

As shown in Fig. 4, the hardware configuration that company servers 11, 12, and 13 and management apparatus 2 may adopt includes, for example, a CPU (Central Processing Unit) 21, main memory 22, secondary storage 23, and an interface (IF) unit 24, all interconnected via an internal bus.

The CPU 21 executes each instruction contained in the genomic information processing program executed by the information processing apparatus (computer) 20. The main memory 22 is, for example, RAM (Random Access Memory), and temporarily stores various programs, such as the genomic information processing program executed by the information processing apparatus (computer) 20, so that the CPU 21 can process them.

The secondary storage 23 is, for example, an HDD (hard disk drive), and is capable of storing various programs, such as the genomic information processing program executed by the information processing apparatus (computer) 20, for the medium to long term. Various programs, such as the genomic information processing program, can be provided as program products recorded on a non-transitory computer-readable storage medium.

IF unit 24 provides interfaces for the input/output of, for example, company servers 11, 12, and 13, and management apparatus 2.

The information processing apparatus (computer) 20, which employs the hardware configuration described above, implements the functions of company servers 11, 12, and 13 and management apparatus 2 by executing the aforementioned genomic information processing method as a program.

Some or all of the above embodiments may be described as set forth in the following Supplementary Notes, but are not limited thereto.

### [Supplementary Note 1]

A method for processing genomic information, wherein a history of each of a series of processes up to identifying location(s) containing mutations in genomic data contained in a patient's specimen is registered and recorded on a blockchain.

### [Supplementary Note 2]

The method for processing genomic information according to Supplementary Note 1, wherein the series of processes comprises:
a process of sequencing a specimen from the patient to generate read data representing a genomic nucleotide sequence;
a process of generating alignment information for the read data by mapping the read data to a reference sequence(s) of the genome(s);
a process of evaluating quality of the alignment information;
a process for detecting variant information from the alignment information to generate variant information.

### [Supplementary Note 3]

The method for processing genomic information according to Supplementary Note 1 or Supplementary Note 2, wherein the history comprises a hash value or a combination of hash values corresponding to a combination of a specimen ID, a patient ID, a processing date, a processing program name, a program version, and processing results (output data, evaluation results).

### [Supplementary Note 4]

A genomic information processing system comprising:
a company server that performs a series of processes up to identify location(s) containing mutations in genomic data contained in a patient's specimen, and issues instructions to register and record a history of each of the processes on a blockchain; and
a management apparatus that receives the instructions from the company server and registers and records the history of each of the processes on the blockchain.

### [Supplementary Note 5]

The genomic information processing system according to Supplementary Note 4, wherein
the company server is present in plural; and
the series of processes is executed by the plural company servers in a distributed manner.

### [Supplementary Note 6]

The genomic information processing system according to Supplementary Note 4 or Supplementary Note 5, wherein the management apparatus is implemented as part of functionality of the company server.

### [Supplementary Note 7]

The genomic information processing system according to any one of Supplementary Notes 4 to 6, wherein the series of processes comprises:
a process of sequencing a specimen from the patient to generate read data representing a nucleotide sequence of a genome(s);
a process of generating alignment information for the read data by mapping the read data to a reference sequence(s) of the genome(s);
a process of evaluating quality of the alignment information;
a process for detecting variant information from the alignment information to generate variant information.

### [Supplementary Note 8]

The genomic information processing system according to any one of Supplementary Notes 4 to 7, wherein the history comprises a hash value or a combination of hash values corresponding to a combination of a specimen ID, a patient ID, a processing date, a processing program name, a program version, and processing results (output data, evaluation results).

### [Supplementary Note 9]

A genomic information processing program that causes a computer to perform processes of registering and recording a history of each of a series of processes on a blockchain, upon receiving instructions from a company server, which performs the series of processes up to identify location(s) containing mutation(s) in genomic data contained in a patient's specimen, to register and record the history of each of the series of processes on the blockchain.

### [Supplementary Note 10]

The genomic information processing program according to Supplementary Note 9, wherein the series of processes comprises:
a process of sequencing a specimen from the patient to generate read data representing a nucleotide sequence(s) of a genome(s);
a process of generating alignment information for the read data by mapping the read data to a reference sequence of the genome;
a process of evaluating quality of the alignment information;
a process for detecting variant information from the alignment information to generate variant information.

Further, the disclosure of Patent Literature cited above is incorporated herein in its entirety by reference thereto. It is to be noted that it is possible to modify or adjust the example embodiments or examples within the scope of the whole disclosure of the present invention (including the Claims) and based on the basic technical concept thereof. Further, it is possible to variously combine or select (or partially omit) a wide variety of the disclosed elements (including the individual elements of the individual claims, the individual elements of the individual example embodiments or examples, and the individual elements of the individual figures) within the scope of the whole disclosure of the present invention. That is, it is self-explanatory that the present invention includes any types of variations and modifications to be done by a skilled person according to the whole disclosure including the Claims and the technical concept of the present invention. Particularly, any numerical ranges disclosed herein should be interpreted that any intermediate values or subranges falling within the disclosed ranges are also concretely disclosed even without specific recital thereof. In addition, using some or all of the disclosed matters in the literatures cited above as necessary, in combination with the matters described herein, as part of the disclosure of the present invention in accordance with the object thereof shall be considered to be included in the disclosed matters of the present application.

### REFERENCE SIGNS LIST

2: management apparatus
3: blockchain
4: genomic database
10: genomic information processing system
11, 12, 13: company servers
20: information processing apparatus (computer)
21: CPU (Central Processing Unit)
22: main memory
23: secondary storage
24: IF (interface) unit

## Claims

1. A method for processing genomic information, wherein a history of each of a series of processes up to identifying location(s) containing mutation(s) in genomic data contained in a patient's specimen is registered and recorded on a blockchain.

2. The method for processing genomic information according to claim 1, wherein the series of processes comprises:
a process of sequencing a specimen from the patient to generate read data representing a genomic nucleotide sequence;
a process of generating alignment information for the read data by mapping the read data to a reference sequence(s) of the genome(s);
a process of evaluating quality of the alignment information;
a process for detecting variant information from the alignment information to generate variant information.

3. The method for processing genomic information according to claim 1 or claim 2, wherein the history comprises a hash value or a combination of hash values corresponding to a combination of a specimen ID, a patient ID, a processing date, a processing program name, a program version, and processing results (output data, evaluation results).

4. A genomic information processing system, comprising:
a company server that performs a series of processes up to identify location(s) containing mutation(s) in genomic data contained in a patient's specimen, and issues instructions to register and record a history of each of the processes on a blockchain; and
a management apparatus that receives the instructions from the company server and registers and records the history of each of the processes on the blockchain.

5. The genomic information processing system according to claim 4, wherein
the company server is present in plural; and
the series of processes is executed by the plural company servers in a distributed manner.

6. The genomic information processing system according to claim 4 or claim 5, wherein the management apparatus is implemented as part of functionality of the company server.

7. The genomic information processing system according to any one of claims 4 to 6, wherein the series of processes comprises:
a process of sequencing a specimen from the patient to generate read data representing a nucleotide sequence of a genome(s);
a process of generating alignment information for the read data by mapping the read data to a reference sequence(s) of the genome(s);
a process of evaluating quality of the alignment information;
a process for detecting variant information from the alignment information to generate variant information.

8. The genomic information processing system according to any one of claims 4 to 7, wherein the history comprises a hash value or a combination of hash values corresponding to a combination of a specimen ID, a patient ID, a processing date, a processing program name, a program version, and processing results (output data, evaluation results).

9. A genomic information processing program that causes a computer to perform processes of registering and recording a history of each of a series of processes on a blockchain, upon receiving instructions from a company server, which performs the series of processes up to identify location(s) containing mutation(s) in genomic data contained in a patient's specimen, to register and record the history of each of the series of processes on the blockchain.

10. The genomic information processing program according to claim 9, wherein the series of processes comprises:
a process of sequencing a specimen from the patient to generate read data representing a nucleotide sequence(s) of a genome(s);
a process of generating alignment information for the read data by mapping the read data to a reference sequence of the genome;
a process of evaluating quality of the alignment information;
a process for detecting variant information from the alignment information to generate variant information.
